# EUROPEAN PATENT APPLICATION

(11) **EP 4 692 799 A2**
(43) Date of publication of application: **11.02.2026**
(21) Application number: 25201470.9
(22) Date of filing: 01.04.2016
(51) Int. Cl.: G01N 33/543

(54) **DETECTION OF STRUCTURAL FORMS OF PROTEIN**

(30) Priority: 21.10.2015 GB 201518675
(62) Divisional of application: 16717579.3
(71) Applicant: Pharmakure Limited, Manchester M13 9NT (GB)
(72) Inventor: STANLEY, Christopher, Liverpool L3 5TF (GB)
(74) Representative: Bartle Read

(57) **Abstract**

The present invention relates to a method of detecting one or more tissue-derived aggregated proteins in a biological sample.

## Description

The present invention relates to a method of detecting and analysing structural forms of proteins in a biological sample as part of a process to diagnose a neurological disease.

In the field of neurodegenerative diseases such as the dementias or Parkinson's Disease there is a particular need to detect the early onset and development of these diseases. Currently the early stages of the major dementias or Parkinson's Disease are difficult to diagnose and stratify correctly and so the treatment prescribed, even if it were the correct choice, often begins too late in the progress of the disease; leading to a poor outcome.

The term dementia covers a large class of neurodegenerative disorders with different causes and overlapping symptoms, of which Alzheimer's disease accounts for 62% of human cases. Each disorder benefits from bespoke management and treatment by clinicians, family and carers, but accurate diagnosis and classification is difficult and is usually based on subjective observations. Another brain neurodegenerative dementia is Huntington's Disease, whilst the general area of neurodegenerative disease can also include systemic forms; an example is amyotrophic lateral sclerosis (ALS) which results from degeneration of the upper and lower motor neurones.

There is a need to stratify neurological diseases such as the dementias and Parkinson's Disease in order to provide early stage treatment and care. A misdiagnosis can result in a patient being treated for the wrong disease with serious long-term consequences.

Currently a patient presenting in clinic is assessed using a range of neurological, cognitive and memory tests. It can be difficult to stratify initially similar diseases such as Parkinson's Disease, Parkinson's with Dementia, Dementia with Lewy Bodies and Alzheimer's Disease.

It is known that aggregates of different types of proteins form in the brain during the progress of a neurodegenerative disease such as Alzheimer's or Parkinson's and they may be involved in the pathogenesis of the disease. The detection of plaques, Lewy bodies or tangles composed of aggregated proteins in the brain by techniques such as imaging in a living subject or by post-mortem histology is considered to be a confirmation of the presence of the specific neurological disease. It is likely that detection of the early stages of protein aggregation will allow intervention with disease modifying treatments before irreversible neurological damage occurs. The content of brain-derived proteins in cerebrospinal fluid (CSF), such as beta-amyloid (also referred to as Abeta) and tau can aid in the diagnosis and stratification of the relevant dementia diseases. For example the Abeta content of CSF is a promising biomarker in differentiating early AD from normal aging processes as well as allowing prediction of those patients with mild cognitive impairment (MCI) who will convert later to Alzheimer's (The Amyloid-Oligomer Count in Cerebrospinal Fluid is a Biomarker for Alzheimer's Disease. Wang-Dietricha, L, Journal of Alzheimer's Disease 34 (2013) 985-994, DOI 10.3233/JAD-122047, which is incorporated herein by reference). A paper reporting on a 7-year study showed that the levels of Abeta, total tau and phosphorylated tau in CSF, as measured by standard immunoassays, could be used to predict the onset both of Alzheimer's Disease and its severity (Amyloid imaging and CSF biomarkers in predicting cognitive impairment up to 7.5 years later. Roe et al, Neurology 2013; 80; 1784-1791, which is incorporated herein by reference).

Since CSF sample collection is inconvenient, often painful and cannot easily be performed on a repeat basis it is not a suitable procedure for routine diagnosis and screening. Biomarker proteins have been reported to be present in blood but at much lower concentrations, which are at the limit of measurement by the standard immunoassay instruments currently available in clinical biochemistry laboratories. Advanced measurement techniques using ultrasensitive immunoassays have suggested that the Abeta peptide in blood may be a reliable indicator of the initial development and progression of Alzheimer's disease. As much as 50% of the monomeric Abeta in blood has been reported to be associated with blood cells and therefore will not be measured by typical immunoassay protocols, which discard the cellular fraction and only test serum or plasma (Reliable Measurements of the Beta-Amyloid Pool in Blood Could Help in the Early Diagnosis of AD. Pesini, P, International Journal of Alzheimer's Disease. Volume 2012, Article ID 604141, doi:10.1155/2012/604141, which is incorporated herein by reference). WO 2011/070174, which is incorporated herein by reference, reporting the same data, describes the use of protein solubilising agents and chaotropic agents to displace, disrupt and solubilize all forms of Abeta from cell surfaces or protein complexes in the cellular fraction of blood or the plasma. The solubilising agent, which is preferably a non-ionic or ionic detergent, is said to be capable of increasing protein solubility, preventing non-specific associations and eliminating inter- and intramolecular aggregation between protein molecules. Thus, in WO 2011/070174 the stated aim is to use detergents and chaotropic agents such as urea to remove all forms of protein interactions and aggregates in blood fractions and to detect the Abeta protein in monomeric form only using a high sensitivity immunoassay. Another study using the highly sensitive assay technique of surface enhanced laser desorption time of flight (SELDI-TOF) mass spectrometry has been used to show that higher concentrations of Abeta dimers are present in the blood of some Alzheimer's patients and the dimers are associated with the membranes of blood cells (Blood-Borne Amyloid-Dimer Correlates with Clinical Markers of Alzheimer's Disease. Villemagne, V, et al. The Journal of Neuroscience, May 5, 2010, 30(18): 6315- 6322, which is incorporated herein by reference) and also described in US 2011/0263450, which is incorporated herein by reference. The analytical protocol involves centrifuging the blood sample to prepare the cellular fraction, which is then subjected to freezing at -80°C, followed by addition of the chaotropic agent urea to a final concentration of 4M, then a dilution of 1:10 in 0.5% Triton X-100 and finally sonication for 15 min on ice. This treatment protocol was reported to break up any potential protein/protein or protein/membrane interactions involving any Abeta moieties, which were then captured using a specific Abeta monoclonal antibody immobilised to a glass slide surface. The presence of captured Abeta monomers and dimers on the surface was then demonstrated with the SELDI-TOF mass spectrometry procedure.

It has been reported that, in another neurodegenerative disease involving protein aggregation (a prion disease) the PrP^{sc} protein, a misfolded and aggregated form of the prion protein is associated with B cells in the blood of scrapie-infected animals (PrPSc is associated with B cells in the blood of scrapie-infected sheep, Edwards et al (Virology 405 (2010) 110-119), which is incorporated herein by reference.

WO2011057029 and WO2009134942 describe a process for detection of protein aggregates from a tissue sample where the preferred method is to capture aggregates first with an immobilized peptide reagent; then to dissociate the aggregates by adding salt such as 0.4 to 1.0M guanidinium HCl or guanidinium SCN and then to detect the eluted material using an ELISA that does not have specificity for the aggregated forms. Alternative harsher dissociation and denaturation conditions, including pH <2.0 or pH >12 or 3-6 M guanidine HCl, are also described that lead to the eluate containing only the monomeric form of the protein. In WO2009134942 it is stated that a buffer of pH 2.3 will dissociate protein aggregates into monomers. In WO2011057029 and WO2009134942 the methods do not capture all structural forms of the protein in the first step.

Other biomarkers have also been studied as indicators of neurological disease. The plasma levels of various forms of α-synuclein, including oligomers, have been shown to have potential value as a diagnostic tool in Parkinson's Disease (A longitudinal study on α-synuclein in blood plasma as a biomarker for Parkinson's disease, Foulds P et al Scientific Reports 3, Article number: 2540 doi:10.1038/srep02540, which is incorporated herein by reference). Tokuda et al have reported on the presence of oligomers of Abeta in serum from non-demented subjects, although the authors suggest that their assay technique may also be detecting non-pathological Aβ complexes associated with serum carrier proteins (Correlation of Aβ oligomer levels in matched cerebrospinal fluid and serum samples, Tokuda et al, Neurosci Lett. 2013 Sep 13;551:17-22. doi: 10.1016/j.neulet.2013.06.029. Epub 2013 Jun 27). Thus not all brain-derived aggregated biomarker proteins present in blood are entirely cell-associated.

Fiandaca et al describe the analysis of neurally-derived exosomes in plasma or serum for the presence of total tau protein, two forms of phosphorylated tau and Abeta 1-42. Detection of these exosome-associated proteins may be useful in the diagnosis of Alzheimer's Disease (Identification of preclinical Alzheimer's disease by a profile of pathogenic proteins in neurally derived blood exosomes: A case-control study (Fiandaca, M, et al Alzheimer's & Dementia 2014 1-8.

Other aggregated proteins involved in the pathogenesis of a neurodegenerative disease include huntingtin in Huntington's Disease and superoxide dismutase (SOD) in ALS.

According to an aspect of the present invention, there is provided a method of detecting and/or analysing the structural forms of a protein in a biological sample comprising:
i. binding some or all of the structural forms of the protein to a surface
ii. eluting at least a portion of the structural forms of the protein from the surface using conditions that reverse the binding interaction;
iii. detecting one or more structural forms of the protein; and optionally,
iv. analysing the content of the different structural forms of the protein in the eluate
wherein the structural forms analysed in step iv) include total protein; monomeric forms; oligomeric forms; multimeric forms and aggregated forms.

According to another aspect of the present invention, there is provided a method of detecting and/or analysing the structural forms of a protein in a biological sample comprising:
i) contacting the sample with a binding agent capable of binding to the structural forms of the protein wherein the forms comprise any of the following: monomer, oligomer; multimer and aggregates;
ii) subjecting the binding agent-protein complex to conditions that are capable of eluting at least a portion of the bound protein forms; and
iii) detecting any two or more of the monomer, oligomer; multimer and aggregate forms of the protein in the eluate

Suitably, the eluting conditions are those which maintain at least a portion of the oligomer; multimer and aggregate forms intact.

Suitably, the eluting step elutes at least a portion of two or more of the bound protein forms.

According to another aspect of the present invention, there is provided a method of detecting and/or analysing the structural forms of a protein in a biological sample comprising:
i) contacting the sample with a binding agent capable of binding to the structural forms of the protein wherein the forms comprise any of the following: oligomer; multimer and aggregates;
ii) subjecting the binding agent-protein complex to conditions that are capable of eluting at least a portion of the bound protein forms; and
iii) detecting any one or more of the oligomer; multimer and aggregate forms of the protein in the eluate .

According to another aspect of the present invention, there is provided a method of detecting and/or analysing the structural forms of a protein in a biological sample comprising:
i) contacting the sample with a binding agent capable of binding to the structural forms of the protein wherein the forms comprise any of the following: oligomer; multimer and aggregates;
ii) subjecting the binding agent-protein complex to conditions that are capable of eluting at least a portion of the bound protein forms; and
iii) detecting any two or more of the monomer, oligomer; multimer and aggregate forms of the protein in the eluate

The total amount of all structural forms present of a specific protein in the sample, or the total amount of aggregated forms or separately the amount of monomeric, oligomeric, multimeric, and aggregate fractions of the protein can be measured individually or collectively by the method and optionally the ratios of the content of these various forms may be calculated. The method of the invention provides an indication of the early stages and subsequent development of a protein conformational disease.

In an embodiment, the biological sample comprises a blood sample. The blood sample may be whole blood or a blood fraction such as serum, plasma or the cellular fraction, including the red cell fraction and/or the white cell fraction and/or the platelets.

In the sample, oligomeric forms of proteins may range in size from dimers up to structures containing 10 to 20 units, multimeric associations of protein subunits may contain from 20 to 100 units, whilst aggregates can be defined as larger structures that can include hundreds, or even thousands of protein molecules. Monomeric, oligomeric, multimeric and aggregated forms in blood may be soluble or associated with endosome, erythrocyte, leukocyte or platelet cell membranes. The proteins that form the oligomers and aggregates may be in a native, normally folded form or they can be misfolded or abnormal in structure and therefore may be more prone to aggregation. Thus, in summary, the forms of the protein present in the sample may comprise soluble monomeric, soluble oligomeric, soluble multimeric, soluble aggregated, cell-associated monomeric, cell-associated oligomeric, cell-associated multimeric and cell-associated aggregated; wherein these species may also include the normally folded or the misfolded forms of the protein or both.

According to another aspect of the invention, there is provided a method of detecting and/or analysing the structural forms of a protein in a biological sample comprising:
i) contacting the sample with a binding agent capable of binding to the structural forms of the protein wherein the forms comprise any two or more of the following: monomer, oligomer; multimer and aggregates;
ii) subjecting the binding agent-protein complex to conditions that are capable of eluting at least a portion of the bound protein forms and which maintain at least a portion of the oligomer; multimer and aggregated forms intact, wherein the pH of the eluting conditions is <4 or >10; and
iii) detecting any two or more of the monomer, oligomer; multimer or aggregate forms of the protein in the eluate

According to another aspect of the invention, there is provided a method of detecting and/or analysing the structural forms of a protein in a biological sample comprising:
i) contacting the sample with a binding agent capable of binding to the structural forms of the protein wherein the forms comprise any one or more of the following: oligomer; multimer and aggregates;
ii) subjecting the binding agent-protein complex to conditions that are capable of eluting at least a portion of the bound protein forms and which maintain at least a portion of the oligomer; multimer and aggregated forms intact, wherein the pH of the eluting conditions is <4 or >10; and
iii) detecting any one or more of the oligomer; multimer or aggregate forms of the protein in the eluate

The binding agent may be an antibody. The binding agent may be immobilised to a surface either by passive adsorbtion e.g. to a hydrophobic or charged surface, or bound covalently via a chemical reaction between reactive moieties on the surface and suitable groups on the binding agent such as amines or thiols. The binding agent may comprise any one or more of the following: an antibody and/or antigen-binding fragment thereof, an aptamer, a lectin, an affibody or a synthetic mimic of an antibody.

Suitably, the surface is the outer surface and interior of a porous bead formed from a cross-linked hydrogel such as agarose or dextran. The beads may have a diameter from 20 to 150 µm and/or the beads may comprise 1% to 6% agarose and/or the beads may have a protein fractionation range from 10,000 daltons to 150,000,000 daltons. A suitable surface on which the specific capture agents may be immobilised in step i) of the method is a bead. The bead may have a diameter between 1 µm to 200 µm. The specific capture agent such as the antibody or ligand may be bound on the surface of the bead, and/or throughout the structure of the bead. The bead is suitably partly formed from a hydrogel.

The inventors have found that the use of large, dense immunocapture beads is highly beneficial when binding proteins from whole blood. It has been observed that during the extended, agitated incubation periods with the beads (which are required to ensure high efficiency of protein capture) the whole blood sample coagulates and becomes a viscous clotted solution. This has been observed even in whole blood samples that have been frozen and thawed and treated with anti-coagulents and detergent solutions. As reported in the Examples the large capture beads can still be separated from whole blood after this immunocapture step whilst the much smaller (<5µm) commercially available beads from suppliers such as Dynal are difficult to recover from the viscous clotted solution.

Other surfaces may also be used in the method, for example the solid surface of a polystyrene microwell may be coated with an antibody specific for a protein. A surface with large pores such as a membrane may also be employed, in this case the cellular component of whole blood will pass through the membrane leaving the protein bound to the protein-specific immobilised antibody.

In the method of the present invention the immunoaffinity capture of the biomarker protein in step i) may be optimised by adjusting sample dilution and buffer composition. The binding conditions may include the use of detergents such as SDS and/or TWEEN, which the inventors believe increases the accessibility of the protein to the binding agent particularly if it is bound to the membranes of blood cells. Alternatively compounds such as the retro-inverso peptide RI-OR2-TAT can be used which is known to bind with high affinity to Abeta peptides and so may act as a displacing agent to increase accessibility to the binding agent (Parthsarathy V et al, A Novel Retro-Inverso Peptide Inhibitor Reduces Amyloid Deposition, Oxidation and Inflammation and Stimulates Neurogenesis in the APPswe/PS1ΔE9 Mouse Model of Alzheimer's Disease, DOI: 10.1371/journal.pone.0054769.

The inventors have demonstrated that adding these solubilizing and displacing agents does not result in total loss of the aggregated forms of the protein as would be expected from the teaching of WO 2011/070174, which is incorporated herein by reference. An advantage of the affinity binding step is that it allows a large blood sample volume to be processed in step 1), i.e. >1 ml, preferably >5 ml or >10 ml, thereby effecting a substantial concentration of the biomarker protein of interest if it is eluted subsequently in step 2) in a small volume, preferably in the range of 50µl to 200 µl.

Surprisingly, in step ii) the inventors have found that use of eluting conditions having a pH adjusted to <4 or >10 causes elution of the bound protein from the binding agent whilst retaining at least a portion of the oligomeric; multimeric and aggregated forms intact as determined in a subsequent assay which is specific for said forms.

In an embodiment, the pH of the eluting conditions is between 1.5 and 4. Suitably, 2 to 4. The eluting conditions may comprise a pH of 2.8. In an embodiment, the pH of the eluting conditions is between 10 and 14. Suitably, 11 to 13. The eluting conditions may comprise a pH of 11.5.

Suitably, a proportion or substantially all of the bound protein is eluted in step ii). Where the elution conditions include either low or high pH incubation the eluate may be neutralised by addition of alkali or acid as appropriate. Other elution conditions employed could include the use of chaotropic salts such as 4M urea, 6M guanidine hydrochloride or >1M potassium iodide. However these conditions are not readily reversible and would be expected to interfere with the subsequent protein aggregate detection step. Alternatively, high concentrations (>5% w/v) of ionic detergents such as SDS or CTAB may be used or organic solvents may also be employed, for example buffers containing alcohols such as ethanol, butanol or isopropanol at 1 to 50% (v/v) provided in all cases that there is no or little interference with subsequent analytical steps. Elution may also be achieved at elevated temperatures eg. 40°C in combination with low or high pH conditions.

Different structural forms of the protein may be recovered from the surface by varying the elution conditions eg. separation of monomer from oligomers, multimers and aggregates can be achieved by using selective elution conditions, for example increasing ionic strength, refining the pH range employed or varying the temperature.

Detection and analysis of eluted protein forms in step iii) may comprise immunological techniques such as immunometric assay configurations where the antibodies employed have either the same epitope specificity or bind specifically to different epitopes or to aggregated forms of a protein. These detection methods may be in the form of solid phase ELISAs or they may be based on biosensor platforms employing physical measurement principles such as surface plasmon resonance. Immunometric methods may also include synthetic ligands with binding specificity for monomeric or aggregated proteins. Other detection methods for monomeric and aggregated proteins include mass spectrometry such as MALDI-TOF and SELDI-TOF techniques designed to detect and quantitate protein monomers, oligomers, multimers and aggregates. The aim of the detection and analysis steps is to determine the content of total protein, the monomeric, oligomeric, multimeric, or aggregated forms individually or in total which are present after elution from the surface; thus as stated above the elution step suitably preserves at least a proportion of the aggregated forms for subsequent detection. In the detection step an immunoassay may be configured to have specificity for all the various forms of the biomarkers in the eluate as described above to provide a measurement of total protein present including all aggregate forms, or it may be configured to have selectivity for two or more of the different forms such as the monomeric, oligomeric, multimeric, or aggregated forms. US 7659076, incorporated herein by reference, describes the use of polymeric ligands in an immunoassay that bind the aggregated forms of proteins under selective conditions and may be used for the detection step of the method.

The proteins detected may comprise beta-amyloid, alpha synuclein (ASN), tau, phosphorylated tau, prion protein, huntingtin, amylin and superoxide dismutase (SOD).

The present invention may be used to detect the presence or absence of a neurodegenerative disease, or any disease giving rise to the oligomerisation and aggregation of a protein as described above where the various forms of said protein are present in blood or another sample type.

Suitably, the methods of the present invention may be used to detect the presence or absence of any one or more neurodegenerative diseases in the group comprising: Alzheimer's Disease (AD), Parkinson's Disease (PD), Huntington's Disease, Dementia with Lewy Bodies, Multiple System Atrophy (MSA), Progressive Supranuclear Palsy, Amyotrophic Lateral Sclerosis (ALS) or Epilepsy.

Accordingly, in a further aspect of the present invention, there is provided a method to assist in the diagnosis of neurological diseases which comprises:
i) determining the content of three or more of Abeta, alpha synuclein (ASN), tau or phosphoprylated tau in a blood sample
ii) defining a sample with elevated beta-amyloid, low ASN and elevated tau or phosphoprylated tau as indicative of Alzheimer's Disease, or
iii) defining a sample with low beta-amyloid, elevated ASN and low tau or phosphoprylated tau as indicative of Parkinson's Disease, or
iv) defining a sample with elevated beta-amyloid, elevated ASN, elevated tau or phosphoprylated tau as indicative of Dementia with Lewy Bodies

In a simplification of this further aspect of the present invention, there is provided a method to assist in the diagnosis of neurological diseases which comprises:
i) determining the content of Abeta or alpha synuclein (ASN in a blood sample
ii) defining a sample with elevated beta-amyloid and low ASN as indicative of Alzheimer's Disease, or
iii) defining a sample with low beta-amyloid and elevated ASN as indicative of Parkinson's Disease, or
iv) defining a sample with elevated beta-amyloid and elevated ASN as indicative of Dementia with Lewy Bodies

In the method "elevated" indicates a level that is significantly above the normal baseline content in blood for age matched controls and "low" means the same level as the age matched controls. "Significantly" may be defined as a level significantly above a normal baseline content which is preferably 2.5 times the standard deviation on the mean of the normal baseline content.

The term "antibody" refers to an immunoglobulin or fragment thereof, and encompasses any polypeptide comprising an antigen-binding fragment or an antigen-binding domain. The term includes but is not limited to polyclonal, monoclonal, monospecific, polyspecific, non-specific, humanized, human, single-chain, chimeric, synthetic, recombinant, hybrid, mutated, grafted, and in vitro generated antibodies. Unless preceded by the word "intact", the term "antibody" includes antibody fragments such as Fab, F(ab')2, Fv, scFv, Fd, dAb, and other antibody fragments that retain antigen-binding function. Typically, such fragments would comprise an antigen-binding domain.

The terms "antigen-binding domain" and "antigen-binding fragment" refer to a part of an antibody molecule that comprises amino acids responsible for the specific binding between antibody and antigen. The part of the antigen that is specifically recognized and bound by the antibody is referred to as the "epitope." An antigen-binding domain may comprise an antibody light chain variable region (VL) and/or an antibody heavy chain variable region (VH). Fd fragments, for example, have two VH regions and often retain some antigen-binding function of the intact antigen-binding domain. Examples of antigen-binding fragments of an antibody include a Fab fragment, a monovalent fragment having the VL, VH, CL and CH1 domains; a F(ab')2 fragment, a bivalent fragment having two Fab fragments linked by a disulfide bridge at the hinge region; a Fd fragment having the two VH and CH1 domains; a Fv fragment having the VL and VH domains of a single arm of an antibody; a dAb fragment (Ward et al., (1989) Nature 341 :544-546), which has a VH domain; an isolated complementarity determining region (CDR), and a single chain Fv (scFv). Although the two domains of the Fv fragment, VL and VH are coded for by separate genes, they can be joined, using recombinant methods, by a synthetic linker that enables them to be made as a single protein chain in which the VL and VH regions pair to form monovalent molecules (known as single chain Fv (scFv); see, for example, Huston et al. ((1988) Proc. Natl. Acad. ScL USA 85:5879-5883). These antibody fragments are obtained using conventional techniques known to those with skill in the art, and the fragments are evaluated for function in the same manner as are intact antibodies.

The term "isolated" refers to a molecule that is substantially free of its natural environment. For instance, an isolated protein is substantially free of cellular material or other proteins from the cell or tissue source from which it was derived. The term also refers to preparations where the isolated protein is sufficiently pure for pharmaceutical compositions; or at least 70-80% (w/w) pure; or at least 80-90% (w/w) pure; or at least 90-95% pure; or at least 95%, 96%, 97%, 98%, 99%, or 100% (w/w) pure.

The terms "specific binding" or "specifically binds" refers to two molecules forming a complex that is relatively stable under physiologic conditions. Specific binding is characterized by a high affinity and a low to moderate capacity as distinguished from nonspecific binding which usually has a low affinity with a moderate to high capacity. Typically, binding is considered specific when the association constant KA is higher than 10<6>M<'1>. If necessary, nonspecific binding can be reduced without substantially affecting specific binding by varying the binding conditions. The appropriate binding conditions, such as concentration of antibodies, ionic strength of the solution, temperature, time allowed for binding, concentration of a blocking agent (e.g., serum albumin, milk casein), etc., may be optimized by a skilled artisan using routine techniques.

Numerous methods known to those skilled in the art are available for obtaining antibodies or antigen-binding fragments thereof. For example, antibodies can be produced using recombinant DNA methods (U.S. Patent 4,816,567). Monoclonal antibodies may also be produced by generation of hybridomas (see e.g., Kohler and Milstein (1975) Nature, 256: 495-499) in accordance with known methods. Hybridomas formed in this manner are then screened using standard methods, such as enzyme-linked immunosorbent assay (ELISA) and surface plasmon resonance (BIACORE(TM)) analysis, to identify one or more hybridomas that produce an antibody that specifically binds with a specified antigen. Any form of the specified antigen may be used as the immunogen, e.g., recombinant antigen, naturally occurring forms, any variants or fragments thereof, as well as antigenic peptide thereof.

One method of making antibodies includes screening protein expression libraries, e.g., phage or ribosome display libraries. Phage display is described, for example, in Ladner et al., U.S. Patent No. 5,223,409; Smith (1985) Science 228:1315-1317; Clackson et al. (1991) Nature, 352: 624-628; Marks et al. (1991) J. Mol. Bio!., 222: 581-597.

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by Morrison (1985) Science 229:1202-1207; by Oi etal. (1986) BioTechniques 4:214; and by US 5,585,089; US 5,693,761; US 5,693,762; US 5,859,205; and US 6,407,213. Those methods include isolating, manipulating, and expressing the nucleic acid sequences that encode all or part of immunoglobulin Fv variable domains from at least one of a heavy or light chain. Such nucleic acids may be obtained from a hybridoma producing an antibody against a predetermined target, as described above, as well as from other sources. The recombinant DNA encoding the humanized antibody molecule can then be cloned into an appropriate expression vector.

For additional antibody production techniques, see Antibodies: A Laboratory Manual, eds. Harlow et al., Cold Spring Harbor Laboratory, 1988. The present invention is not necessarily limited to any particular source, method of production, or other special characteristics of an antibody.

Antibodies, also known as immunoglobulins, are typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgGi, IgG2, IgG[beta], IgG-i, IgAi, and IgA2. Each light chain includes an N-terminal variable (V) domain (VL) and a constant (C) domain (CL). Each heavy chain includes an N-terminal V domain (VH), three or four C domains (CHs), and a hinge region. The CH domain most proximal to VH is designated as CH1. The VH and VL domains consist of four regions of relatively conserved sequences called framework regions (FR1 , FR2, FR3, and FR4), which form a scaffold for three regions of hypervariable sequences (complementarity determining regions, CDRs). The CDRs contain most of the residues responsible for specific interactions of the antibody with the antigen. CDRs are referred to as CDR 1, CDR2, and CDR3. Accordingly, CDR constituents on the heavy chain are referred to as H1, H2, and H3, while CDR constituents on the light chain are referred to as L1 , L2, and L3.

CDR3 is typically the greatest source of molecular diversity within the antibody-binding site. For a review of the antibody structure, see Antibodies: A Laboratory Manual, Cold Spring Harbor Laboratory, eds. Harlow et al., 1988.

The Fab fragment (Fragment antigen-binding) consists of VH-CRI and VL-CL domains covalently linked by a disulfide bond between the constant regions. The Fv fragment is smaller and consists of VH and VL domains non-covalently linked. To overcome the tendency of non-covalently linked domains to dissociate, a single chain Fv fragment (scFv) can be constructed. The scFv contains a flexible polypeptide that links (1) the C-terminus of VH to the N-terminus of VL, or (2) the C-terminus of VL to the N-terminus of VH. A 15-mer (Gly4Ser)3 peptide may be used as a linker, but other linkers are known in the art.

VHH molecules (or nanobodies), as known to the skilled artisan, are heavy chain variable domains derived from immunoglobulins naturally devoid of light chains, such as those derived from Camelidae as described in WO9404678, incorporated herein by reference. Such a VHH molecule can be derived from antibodies raised in Camelidae species, for example in camel, llama, dromedary, alpaca and guanaco and is sometimes called a camelid or camelized variable domain. See e.g., Muyldermans., J. Biotechnology (2001 ) 74(4):277-302, incorporated herein by reference. Other species besides Camelidae may produce heavy chain antibodies naturally devoid of light chain. VHH molecules are about 10 times smaller than IgG molecules. They are single polypeptides and very stable, resisting extreme pH and temperature conditions. Moreover, they are resistant to the action of proteases which is not the case for conventional antibodies. Furthermore, in vitro expression of VHHs produces high yield, properly folded functional VHHs. In addition, antibodies generated in Camelids will recognize epitopes other than those recognized by antibodies generated in vitro through the use of antibody libraries or via immunization of mammals other than Camelids (see WO 9749805, which is incorporated herein by reference).

Once the protein form has been detected, a suitable treatment plan for a subject may follow which may comprise the administration of at least one therapeutic compound.

The present invention will now be described, by way of example only, with reference to the following examples and figures.
Fig. 1 shows the fluorescence measurements from Example 1;
Fig. 2 shows the results from the capture, elution and detection experiment of Example 2;
Fig. 3a shows the results of Example 4 with the fresh set of blood samples from patents diagnosed with AD, control blood samples from spouses (age matched) and a further range of different dementias (MCI - mild cognitive impairment, PD - Parkinson Disease, FTD - fronto temporal dementia, DLB - dementia with lewy bodies, PSP - progressive supranuclear palsy);
Fig. 3b shows the same data in 3a presented as a scattergram;
Fig. 3c shows the ROC curve for the AD results vs. age matched spouse controls, this data is summarised in Table 2;
Fig. 4 shows the results of the aggregated Abeta assay carried out according to the method of Example 4.
Figure 5 shows the results of the assays for aggregated Abeta in whole blood from younger subjects
Figure 6 shows the results of the assay for total Abeta and the aggregated fraction in whole blood.

### Examples

**Example** 1. Investigation into protein solubilising and disrupting reagents that maximise immunocapture of Abeta spiked into whole blood.

Beads derivatized with Protein A (GE Healthcare, magnetic Sepharose, diameter 50 µm to 100 µm) were coated with mouse monoclonal anti Abeta antibody (4G8) by incubating for 30 min at room temperature (r.t.) with a solution of 4G8 in PBS at 40 µg/ml, with gentle agitation. Beads were blocked with a 1% skimmed milk solution for 1 hour. Pooled whole blood from normal controls was spiked at 50 pg/ml with a synthetic Abeta 1-42 peptide which had been labelled with biotin; this solution had previously been shown to contain a proportion of peptide aggregates.

100 µl volumes of blood and antibody-coated beads were mixed overnight at 4°C and were then washed 3x with PBS buffer pH 7.5 containing 0.2% Tween 20. Then immunocaptured Abeta was detected directly on the beads by adding 100 µl of a streptavidin-europium conjugate (Pierce), incubating for 60 min, washing the beads in PBS Tween, displacing europium using the standard dissociation solution and reading the fluorescence signal in the supernatant in a time resolved fluorimeter. During the immunocapture step various protein solubilising and disrupting agents were added to the blood according to the list in Table 1.

**Table 1.**

| Sample | Reagents added |
|---|---|
| A | None |
| B | SDS 0.2% |
| C | RI-OR2-TAT 2nM |
| D | SDS 0.2%, RI-OR2-TAT 2nM |
| E | SDS 0.2%, RI-OR2-TAT 0.2nM |
| F | SDS 0.1%, RI-OR2-TAT 2nM |
| G | Tween 0.05%, SDS 0.15% |
| H | Tween 0.1%, SDS 0.1% |
| I | Tween 0.15%, SDS 0.05% |
| J | None, Dynabeads |

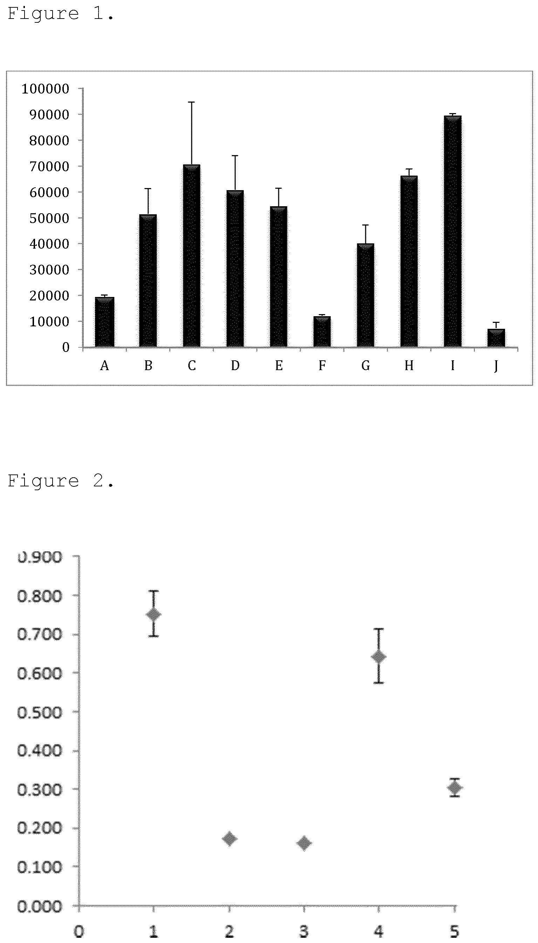

Figure 1 shows the fluorescence measurements. These data indicate that the whole blood matrix was inhibitory to the immunocapture of spiked Abeta peptide (Sample A) whilst recovery was considerably improved by adding either detergents or the peptide displacing agent RI-OR2-TAT (supplied by Lancaster University, UK) that is known to disrupt Abeta interactions. The Tween/SDS mix in reaction I gave the best recovery. (The results for F are an anomaly). In reaction J the large beads from GE Healthcare were replaced by small (1 µm diameter) Protein A-coated magnetic Dynabeads supplied by Invitrogen Inc. These were clearly far less successful in extracting spiked Abeta from the whole blood matrix. There was no additive effect when SDS was combined with RI-OR2-TAT suggesting that they are acting on the same population of Abeta in the blood. Note that this experiment did not discriminate between the monomeric and aggregated forms of Abeta; it simply showed the effect of added reagents on the efficiency of immunocapture of all forms of the spiked protein in the blood matrix.

**Example** 2. Detection of synthetic aggregated Abeta 1-42 peptide spiked into buffer and whole blood.

Beads derivatized with Protein A (GE Healthcare, magnetic Sepharose) were coated with mouse monoclonal anti beta amyloid antibody (4G8) according to Example 1. Abeta 1-42 peptide which had been aggregated previously by incubating at r.t. for 16-24 hours was spiked into PBS or fresh whole blood (1ml) at 100 pg/ml and 50 µl of 4G8-decorated beads were added. The detergents SDS and Tween 20 were added to a final concentration of 0.05% and 0.15% respectively. As demonstrated previously in Example 1 the addition of these detergents improved the recovery of spiked Abeta in the whole blood matrix. The mixture was agitated for 1 hour at r.t. to capture Abeta onto the beads, which were then captured with a magnet and washed six times with PBS buffer to remove the blood matrix. After aspiration of the supernatant 100 µl of glycine-HCl elution buffer pH 2.8 was added and the beads were incubated for 20 min at r.t. to dissociate Abeta from the 4G8 binding agent and most probably to release some or all of the 4G8 antibody from the covalently attached Protein A on the bead. The eluate was neutralised by addition of 200 µl 0.2M-NaOH and transferred to an Abeta aggregate ELISA detection kit supplied by Microsens Biotechnologies (London, UK). The kit has a microplate well coated with a polymeric ligand that is thought to bind all forms of aggregated Abeta and the manufacturers' protocol involves aggregate capture, binding with a different monoclonal antibody with specificity for Abeta (BAM10) and detection with a secondary rabbit anti mouse HRP conjugate. After incubating with TMB substrate for 30 min the reaction was stopped with 0.1M HCl and the absorbance in the wells was measured at 450nm.

Figure 2 shows the results from this capture, elution and detection experiment. In the Figure sample 1 is the positive control in the assay (Abeta solution as supplied by the manufacturer), sample 2 is the negative control (no Abeta), sample 3 is a bead control (no 4G8 antibody added), sample 4 is the Abeta sample at 100 pg/ml spiked into PBS and sample 5 is the Abeta sample at 100 pg/ml spiked into whole blood. It is estimated that, based on previous analytical studies by the manufacturer and described on their website that the aggregate content of this spiked sample was 1% or less, hence the assay was detecting <1 pg/ml aggregated beta amyloid. This small amount of aggregated material was clearly bound by the immobilised 4G8 antibody on the beads and eluted using a brief acid incubation. The matrix effect of whole blood on the assay i.e. inhibition of binding was also still apparent in that the amount of Abeta recovered was significantly reduced compared to PBS alone, but was still measurable. This Example shows that at least a proportion of the aggregated Abeta spiked into blood was carried through the entire procedure of immunoconcentration in the presence of detergents, extensive washing to remove blood components, acid elution from the beads and neutralisation such that sufficient material remained in the eluate to give a signal in the aggregated protein ELISA detection kit.

**Example 3.** Measurement of aggregated Abeta in whole blood from dementia patients using an ultrasensitive time resolved fluorescence method.

Beads derivatized with Protein A (GE Healthcare, magnetic Sepharose) were coated with mouse monoclonal anti beta amyloid antibody (4G8) according to Example 1 and 50 µl of 4G8-decorated beads were then added and incubated with 1 ml of whole blood taken from patients diagnosed as having a neurological disease, and also from healthy age-matched controls. The blood samples had been aliquotted and frozen at -80°C immediately after drawing. Hence they had been subjected to a single freeze/thaw cycle prior to assay. The disrupting reagents SDS and Tween 20 were also added to the thawed blood at a final concentration of 0.05% and 0.15% respectively. The mixture was agitated for 1 hour at room temperature (r.t.) to capture Abeta onto the beads, which were then captured with a magnet and washed six times with 1 mL PBS buffer to remove the blood matrix. After aspiration of the supernatant 100 µl of glycine-HCl elution buffer pH 2.8 was added and the beads were incubated for 20 min at r.t. to dissociate Abeta from the 4G8 binding agent The eluate was neutralised by addition of 200 µl 0.2M-NaOH and transferred to an Abeta aggregate ELISA detection kit supplied by Microsens Biotechnologies (London, UK). The detection antibody in the kit was replaced with an alternative commercially available antibody (designation 6E10) which had been biotinylated. In the revised assay a wash step was used to remove excess biotinylated 6E10 and then a solution of streptavidin-europium conjugate in PBS buffer was added, incubated for 30 min, excess conjugate was removed by further washing and the signal in the microwell was detected after addition of enhancer/dissociation solution (Pierce) using the standard procedure of time resolved fluorescence (TRF) in a microplate fluorimeter. TRF is an analytical method with a much lower detection limit (1000x) for the europium label employed than the conventional enzyme label used in the ELISA described in Example 2.

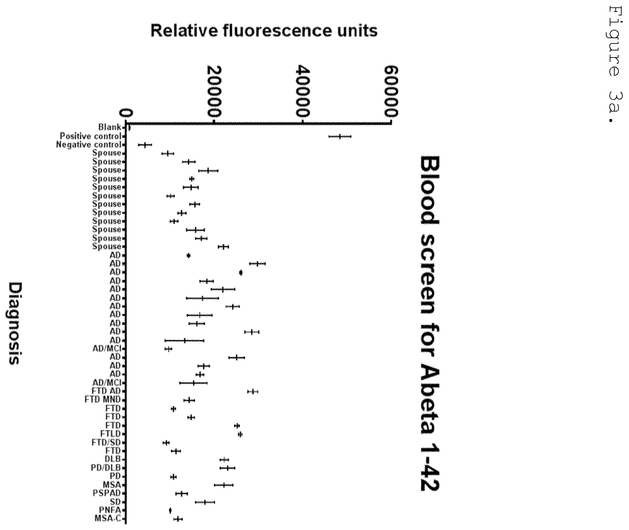

Figure 3a shows the results with the blood samples from patents diagnosed with AD, control blood samples from spouses (age matched) and a further range of different dementias (MCI - mild cognitive impairment, PD - Parkinson Disease, FTD - fronto temporal dementia, DLB - dementia with lewy bodies, PSP - progressive supranuclear palsy. The Student's t-test for spouse vs. Alzheimer's gave a p value of 0.00152 (a highly significant difference) whilst the t-test for Alzheimer's vs. other dementias was 0.264 (no significant difference).

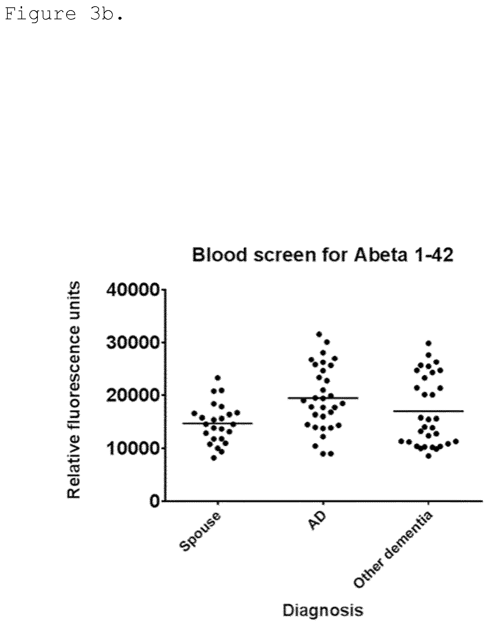

Figure 3b shows the same data in 3a presented as a scattergram.

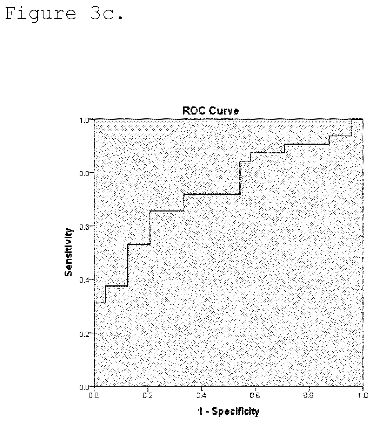

Figure 3c shows the ROC curve for the AD results vs. age matched spouse controls, this data is summarised in Table 2. The AUC of 0.736 represents a fair discrimination between age matched controls and AD patients.

**Table 2.**

| **Area Under the Curve** | | | | |
|---|---|---|---|---|
| | | | | |

| Area | Std. Error^{a} | Asymptotic Sig.^{b} | Asymptotic 95% Confidence Interval | |
|---|---|---|---|---|
| | | | Lower Bound | Upper Bound |
| .736 | .066 | .003 | .606 | .866 |
| a. Under the nonparametric assumption | | | | |
| b. Null hypothesis: true area = 0.5 | | | | |

**Example 4.** Determination of optimal pH elution conditions to release Abeta from capture beads using an ultrasensitive time resolved fluorescence method.

The procedure described in Example 3 was carried out using a solution of aggregated Abeta at 100pg/ml in PBS. A pH range from 1.5 to 4 was investigated to determine the optimal elution conditions for the Abeta peptide from the beads whilst maintaining at least a proportion of the aggregated forms intact.

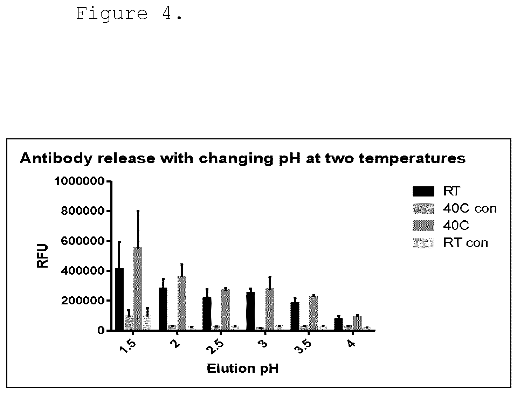

Figure 4 shows that the release of Abeta from the beads is more efficient at lower pH, and is also improved using a higher temperature during elution (40°C). Surprsingly even at the very low pH of 1.5 aggregated Abeta is still detectable and so has not been completely dissociated and denatured under these harsh elution conditions.

**Example 5.** Measurement of aggregated Abeta in whole blood from dementia patients using a double antibody ELISA method.

The procedure described in Example 3 was repeated including the step of elution of captured Abeta from the beads. The subsequent detection procedure was revised however and a double antibody assay method was used instead of the polymeric ligand assay. In this revised assay method the same monoclonal antibody was used on both sides of the ELISA 'sandwich' i.e. the microplate well was coated with 6E10 as the capture antibody and the detection antibody conjugate was biotinylated 6E10. Final detection of bound biotinylated antibody in the microwell was carried out with a streptavidin-europium conjugate and measurements were undertaken as before in a time resolved microplate fluorimeter. This revised assay configuration theoretically detects only the oligomers, multimers and aggregates of Abeta present in blood since no signal would be obtained from the assay if a multi-epitopic version of Abeta was not present (due to epitope masking by the antibody).

Without the prior antibody-coated bead capture step; there was no significant difference in a T-test between age-matched spouse and dementia patients However, using the same blood samples with the antibody-coated bead capture step prior to the double antibody assay method gave a T-test on spouse vs. AD patients of 0.07 which is close to a significant result, thus indicating that the initial antibody-coated bead capture step for Abeta is critical to obtaining a useful assay result.

**Example 6.** Measurement of aggregated tau spiked into whole blood.

Tau protein is a microtubule-associated protein (MAP) that is highly soluble in the cytoplasm. Tauopathies are a class of neurodegenerative diseases associated with the pathological aggregation of tau protein in the brain.

To detect aggregated tau protein in whole blood beads were first coated with anti-tau monoclonal antibody (Ab64193, Abcam) using the method described in Example 1. Tau protein (rPeptide, 4241 Mars Hill Road, Bogart, GA 30622, USA) in PBS buffer was aggregated by the addition of 1.5 µM arachidonic acid (Chirita et al. J Biol Chem. 2003 Jul 11;278(28):25644-50. Anionic micelles and vesicles induce tau fibrillization in vitro). The aggregated tau protein was spiked into a whole blood sample from a healthy volunteer (exhibiting no signs of dementia) at 20 pg/ml. The detergents sarcosyl (0.1% w/v) and Tween 20 (0.1% v/v) were added to the blood sample to enhance recovery of tau protein by the anti-tau antibody coated beads. After incubating for 60 min the beads were separated from the whole blood and washed 6x in PBS buffer. The bound tau protein was then eluted at pH 2.8 as described above for Abeta assays and then aggregated forms were detected in the ligand-based microplate assay described in earlier examples using a second anti-tau antibody (HT7, Thermo Scientific) and a streptavidin-europium conjugate. The results obtained are shown in Table 3.

**Table 3. Detection of aggregated tau protein spiked into whole blood**

| Sample | Relative fluorescence units (RFU) in TRF spectrometer |
|---|---|
| Negative control (no tau aggregate added) | 5000 |
| Positive control (20pg/ml aggregated tau protein added) | 22000 |

**Example** 7. Measurement of aggregated alpha synuclein (ASN) spiked into whole blood.

Alpha-synuclein is abundant in the human brain where it is found mainly in the presynaptic terminals. The protein is involved in maintaining a supply of synaptic vesicles in presynaptic terminals and in regulating the release of the neurotransmitter dopamine.

The assay protocol described in Example 6 was followed with the substitution of anti-ASN monoclonal antibody (sc 211, Santa Cruz) on the beads and biotinylated anti-ASN monoclonal antibody (LB 509, Abcam) as the detection antibody in the ligand-based microplate assay for aggregated proteins. ASN protein (rPeptide) in PBS was aggregated by shaking at 37°C for 5 days, at a protein concentration of 50µM (Foulds et al FASEB J. 2011 Dec;25(12):4127-37. Phosphorylated α-synuclein can be detected in blood plasma and is potentially a useful biomarker for Parkinson's disease). The aggregated tau protein was spiked into a whole blood sample from a healthy volunteer (exhibiting no signs of dementia) at 20 pg/ml. The detergents sarcosyl (0.1% w/v) and Tween 20 (0.1% v/v) were added to the whole blood sample and the pH was lowered to 4 with citrate buffer to enhance recovery of ASN protein by the anti-ASN antibody coated beads. The results obtained in the aggregated protein assay are shown in Table 4.

**Table 4. Detection of aggregated ASN protein spiked into whole blood**

| Sample | Relative fluorescence units (RFU) in TRF spectrometer |
|---|---|
| Negative control (no ASN aggregate added) | 25000 |
| Positive control (20pg/ml aggregated ASN protein added) | 29000 |

**Example** 8. Detection of synthetic aggregated Abeta 1-42 peptide spiked into PBS and whole blood using high pH elution.

The procedure involving capture of aggregated Abeta from whole blood drawn from a normal subject, as described in Example 2, was repeated with a change in elution conditions to pH 11.5 using 0.1M CAPS buffer followed by neutralisation to pH 7 with 0.1M HCl. The results from the aggregated protein assay are shown in Table 5.

**Table 5. Detection of aggregated Abeta protein spiked into whole blood and eluted from the capture beads at pH 11.5**

| Sample | Relative fluorescence units (RFU) in TRF spectrometer (single 1 ml samples) |
|---|---|
| Negative control (no Abeta aggregate added) | 12000 |
| Positive control (20pg/ml aggregated Abeta protein added) | 18000 |

In a further experiment the elution pH was raised to 12.8 using 0.2M KCl/NaOHbuffer and compared to elution at pH 3 using glycine-HCl. Aggregated Abeta was spiked into both PBS buffer and whole blood taken from a normal subject. Table 6 shows the comparison of high pH and low pH elution which, under these conditions, proved to give very similar results. In this experiment 1 ml whole blood or PBS samples were tested in triplicate and the controls used capture beads which had not been coated with the 4G8 Abeta-specific antibody.

**Table 6. Detection of aggregated Abeta protein spiked into whole blood or PBS and eluted from the capture beads at pH 3 and 12.8**

| Sample | Relative fluorescence units (RFU) in TRF spectrometer (mean and SD of 1ml triplicate samples) |
|---|---|
| Assay blank | 5469 |
| | SD=898 |
| PBS | |
| pH 3, control, no capture antibody on beads | 15838 |
| | SD=4630 |
| pH 3, with capture antibody | 176582 |
| | SD=38815 |
| pH 12.8 control, no capture antibody on beads | 16457 |
| | SD=5846 |
| pH 12.8, with capture antibody | 163453 |
| | SD=21515 |

| Blood | |
|---|---|
| pH 3, control, no capture antibody on beads | 44956 |
| | SD=6832 |
| pH 3, with capture antibody | 120661 |
| | SD=36020 |
| pH 12.8 control, no capture antibody on beads | 29545 |
| | SD=819 |
| pH 12.8, with capture antibody | 114882 |
| | SD=58265 |

### Example 9. Levels of aggregated Abeta in young normal controls

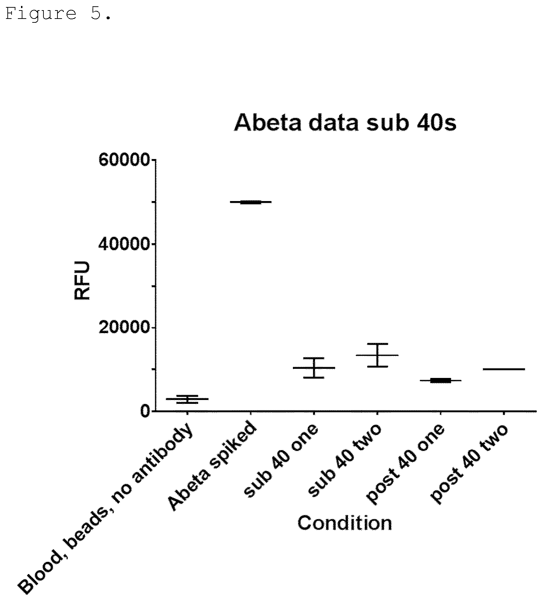

Two pools of whole blood were prepared from normal volunteers, the first group was <40 years and the second >40 years, but < 60. The results of the aggregated Abeta assay carried out according the method of Example 4 are presented in Figure 5. The data indicate that the levels reported in these young normal individuals are in the range of 10,000 to 15,000 RFUs; this is significantly below the mean of age matched (>60 year) controls shown in Figure 3b which indicates that aggregated Abeta levels in the blood increase with age.

**Example 10.** Measurement of the content of total Abeta and total aggregated forms in whole blood using an ELISA.

The first stage of the assay procedure described in Example 3 was carried out using Abeta-specific antibody-coated beads, followed by acid elution at pH 2.8 and neutralisation. Then 100 µl of the neutralised eluate was added to duplicate wells of a commercially available total Abeta 1-42 ELISA kit (Life Technologies) and the manufacturer's recommended protocol was followed. This kit measures total Abeta 1-42 content rather than the aggregated form only. In a further variant of this protocol 200 µl of the neutralised eluate was added to the wells of the aggregate detection kit provided by Microsens Biotechnologies and incubated for 45 min to bind at least a proportion of the total aggregates present (including all the oligomeric forms) under the low ionic strength conditions employed; then the supernatant in the wells was combined and transferred to a single well of the total Abeta 1-42 ELISA kit and a total protein assay was carried out as above. In addition 100 µl of the original whole blood sample was added directly to the wells of the Abeta ELISA and the manufacturer's protocol was followed to determine whether this assay could be used to detect total Abeta in the unprocessed original whole blood sample.

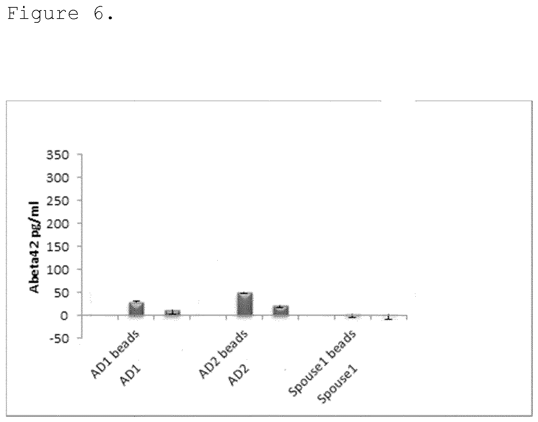

Figure 6 shows the data from the total Abeta measurements in the Life Technologies kit. The results obtained with whole blood added directly to the wells have been omitted as the background levels were very high and variable and no useful data was obtained. However, the results obtained with the neutralised eluate from the bead capture step showed that significant levels of total Abeta 1-42 could be detected in two patients diagnosed with Alzheimer's Disease ("AD 1 beads or AD2 beads"), whilst the age matched (spouse) control level was zero ("Spouse 1 beads"). The Figure shows the mean of duplicate assays in the ELISA and the standard variation. The results from the samples that had been treated with the aggregate-binding microwells prior to the total Abeta 1-42 ELISA showed a reduction of approx. 60% in signal in the ELISA from the AD patients ("AD1", "AD2"). This result provided evidence that the neutralised eluate contained approx. 60% total aggregated and approx. 40% non-aggregated forms of Abeta 1-42 and indicated that determining the content of these forms of the protein could be used to improve the accuracy of a blood test for Alzhiemer's Disease.

A repeat of this experiment using capture conditions of high ionic strength in the aggregate detection kit (which ensures that only larger aggregates and not smaller oligomeric forms are bound) substantially reduced the signal from the AD patients indicating that the oligomeric forms predominated in the whole blood sample
This Example shows that the immunoconcentration step using protein specific antibody-coated capture beads is an efficient way of extracting all structural forms of proteins from whole blood and the neutralised eluate from the bead capture step could be used in a commercial ELISA for total protein (this Example) as well as in the aggregate-specific protein assays (previous Examples).

**Example** 11. Measurement of aggregated Abeta in whole blood and plasma from Alzheimer's Disease patients.

The assay procedure described in Example 3 was carried out to measure aggregated Abeta in 1 ml aliquots of whole blood or 5 ml aliquots of plasma taken from Alzheimer's Disease (AD) patients. Aggregated Abeta can be detected in both sample types, however the data also indicates that levels in plasma were at least 6x lower in plasma than in whole blood suggesting that the majority of the aggregated forms of this protein were associated with the cellular fraction of blood.

**Example 12.** Measurement of aggregated Abeta, aggregated alpha synuclein and aggregated tau in whole blood from dementia and Parkinson's Disease patients.

The method described in Examples 3, 6 and 7 was used to determine the levels of aggregated Abeta, aggregated alpha synuclein and aggregated tau in separate 1 ml aliquots of whole blood taken from Alzheimer's Disease (AD), Dementia with Lewy bodies (DLB) and Parkinson's Disease (PD) patients (8 subjects in total). The assays were conducted in duplicate. The blood samples had previously been aliquotted and frozen at -80°C immediately after drawing. Hence they had been subjected to a single freeze/thaw cycle prior to assay.

Table 6 is a summary of the levels of these three aggregated proteins determined to be present in the blood samples whilst Table 7 shows the expected results from the known levels of large aggregates of these proteins in brain tissue of patients with a specific neurodegenerative disease. It is clear that 100% of the AD patients tested had the expected levels of circulating aggregated proteins present in blood, whilst 66% of the DLB or PD patients had the expected levels of proteins.

**Table 6. Summary of levels of aggregated proteins measured in whole blood.**

| Disease | β-amyloid | α-synuclein | tau |
|---|---|---|---|
| Age matched control (spouse) | Low | Low | Low |
| AD 1 | High | Low | High |
| AD 2 | High | Low | Above control |
| DLB 1 | High | High | High |
| DLB 2 | Low | High | High |
| DLB 3 | High | High | High |
| PD 1 | Low | High | Low |
| PD 2 | Low | High | ND |
| PD 3 | High | High | ND |

**Table 7. Expected values**

| Disease | β-amyloid | α-synuclein | tau |
|---|---|---|---|
| AD | High | Low | High |
| DLB | High | High | High |
| PD | Low | High | Low |

THE FOLLOWING STATEMENTS ARE NOT THE CLAIMS BUT ARE FURTHER ASPECTS OF THE INVENTION:
1. A method of detecting and/or analysing the structural forms of a protein in a biological sample comprising:
   i. binding some or all of the structural forms of the protein to a surface
   ii. eluting at least a portion of the structural forms of the protein from the surface using conditions that reverse the binding interaction
   iii. detecting one or more of the structural forms of the protein; and optionally
   iv. analysing the content of the different structural forms of the protein in the eluate
2. The method of statement 1 wherein the structural forms analysed in step iv) include total protein; monomeric forms; oligomeric forms; multimeric forms and aggregated forms.
3. The method of statement 1 wherein the pH of the eluting conditions is <4 or >10.
4. The method of statement 3 wherein the pH of the eluting conditions is between 1.5 and 4, or 10 and 14.
5. The method of statement 4 wherein the pH of the eluting conditions is between 2 to 4, or 11 to 13.
6. The method of statement 5 wherein the pH of the eluting conditions is 2.8 or 11.5.
7. The method of statement 1 wherein the biological sample comprises whole blood, serum, plasma or the cellular fraction, including the red cell fraction, the white cell fraction and/or the platelets.
8. A method of detecting and/or analysing the structural forms of a protein in a biological sample comprising:
   i. contacting the sample with a binding agent capable of binding to the structural forms of a protein wherein the forms comprise any two or more of the following: monomer, oligomer; multimer and aggregates;
   ii. subjecting the binding agent-protein complex to conditions that are capable of eluting at least a portion of the bound protein forms and which maintain at least a portion of the oligomer; multimer and aggregate forms intact; and
   iii. detecting any two or more of the monomer, oligomer; multimer and aggregate forms of the protein in the eluate
9. The method of any one or more of the previous statements wherein the binding agent is immobilised to a surface.
10. The method of statement 9 wherein the surface is the outer surface and/or interior of a porous bead.
11. The method of statement 10 wherein the beads have a diameter from 20 to 150 µm.
12. The method of statement 10 or 11 wherein the beads may comprise 1% to 6% agarose.
13. The method of any one more of the previous statements wherein protein which has bound and then been eluted is neutralised by addition of alkali or acid.
14. The method of any one or more of the previous statements wherein elution is achieved at a temperature of between 20°C and 40°C.
15. The method of any one or more of the previous statements where the detection method comprises the use of solid phase ELISAs, biosensor platforms, surface plasmon resonance, immunometric methods comprising synthetic ligands with binding specificity for aggregated proteins and/or mass spectrometry.
16. The method of any one of the previous statements wherein the protein species to be detected comprises any one or more of: beta-amyloid, alpha synuclein (ASN), tau, phosphorylated tau, prion protein, huntingtin and SOD.
17. The method of any one or more of the previous statements for detecting the presence or absence of a neurodegenerative disease.
18. The method of any one or more of the previous statements for detecting the presence or absence of any one or more neurodegenerative diseases in the group comprising: Alzheimer's Disease (AD), Parkinson's Disease (PD), Huntington's Disease, Dementia with Lewy Bodies, Multiple System Atrophy, Progressive Supranuclear Palsy, Amyotrophic Lateral Sclerosis (ALS) or Epilepsy.
19. The method of any one or more of the previous statements wherein the binding agent comprises any suitable ligand capable of binding to structural forms of a protein.
20. The method of statement 19 wherein the ligand comprises any one or more of the following: an antibody and/or antigen-binding fragment thereof, an aptamer, a lectin, an affibody or a synthetic mimic of an antibody.
21. A kit of parts for use in the method of any one or more of statements 1 to 20, said kit comprising:
   i) a binding agent capable of binding to the structural forms of a protein;
   ii) an eluting agent capable of eluting at least a portion of the bound protein forms and which maintain at least a portion of the oligomer; multimer and larger aggregate forms intact; and
   iii) a detecting agent for detecting two or more of total protein; monomeric forms; oligomeric forms; multimeric forms and aggregated forms.

## Claims

1. A method of detecting and/or analysing the structural forms of a protein in a biological sample comprising:
i. binding some or all of the structural forms of the protein to a surface
ii. eluting at least a portion of the structural forms of the protein from the surface using conditions that reverse the binding interaction
iii. detecting one or more of the structural forms of the protein; and optionally
iv. analysing the content of the different structural forms of the protein in the eluate

2. The method of claim 1 wherein the structural forms analysed in step iv) include total protein; monomeric forms; oligomeric forms; multimeric forms and aggregated forms.

3. The method of claim 1 wherein the pH of the eluting conditions is <4 or >10.

4. The method of claim 3 wherein the pH of the eluting conditions is between 1.5 and 4, or 10 and 14; or wherein the pH of the eluting conditions is between 2 to 4, or 11 to 13.

5. The method of claim 4 wherein the pH of the eluting conditions is 2.8 or 11.5.

6. The method of claim 1 wherein the biological sample comprises whole blood, serum, plasma or the cellular fraction, including the red cell fraction, the white cell fraction and/or the platelets.

7. A method of detecting and/or analysing the structural forms of a protein in a biological sample comprising:
i. contacting the sample with a binding agent capable of binding to the structural forms of a protein wherein the forms comprise any two or more of the following: monomer, oligomer; multimer and aggregates;
ii. subjecting the binding agent-protein complex to conditions that are capable of eluting at least a portion of the bound protein forms and which maintain at least a portion of the oligomer; multimer and aggregate forms intact; and
iii. detecting any two or more of the monomer, oligomer; multimer and aggregate forms of the protein in the eluate

8. The method of any one or more of the previous claims wherein the binding agent is immobilised to a surface and/or
wherein the surface is the outer surface and/or interior of a porous bead and/or
wherein the beads have a diameter from 20 to 150 µm and/or
wherein the beads may comprise 1% to 6% agarose.

9. The method of any one more of the previous claims wherein protein which has bound and then been eluted is neutralised by addition of alkali or acid and/or
the method of any one or more of the previous claims wherein elution is achieved at a temperature of between 20°C and 40°C.

10. The method of any one or more of the previous claims where the detection method comprises the use of solid phase ELISAs, biosensor platforms, surface plasmon resonance, immunometric methods comprising synthetic ligands with binding specificity for aggregated proteins and/or mass spectrometry.

11. The method of any one of the previous claims wherein the protein species to be detected comprises any one or more of: beta-amyloid, alpha synuclein (ASN), tau, phosphorylated tau, prion protein, huntingtin and SOD.

12. The method of any one or more of the previous claims for detecting the presence or absence of a neurodegenerative disease and/or
for detecting the presence or absence of any one or more neurodegenerative diseases in the group comprising: Alzheimer's Disease (AD), Parkinson's Disease (PD), Huntington's Disease, Dementia with Lewy Bodies, Multiple System Atrophy, Progressive Supranuclear Palsy, Amyotrophic Lateral Sclerosis (ALS) or Epilepsy.

13. The method of any one or more of the previous claims wherein the binding agent comprises any suitable ligand capable of binding to structural forms of a protein.

14. The method of claim 13 wherein the ligand comprises any one or more of the following: an antibody and/or antigen-binding fragment thereof, an aptamer, a lectin, an affibody or a synthetic mimic of an antibody.

15. A kit of parts for use in the method of any one or more of claims 1 to 14, said kit comprising:
i. a binding agent capable of binding to the structural forms of a protein;
ii. an eluting agent capable of eluting at least a portion of the bound protein forms and which maintain at least a portion of the oligomer; multimer and larger aggregate forms intact; and
iii. a detecting agent for detecting two or more of total protein; monomeric forms; oligomeric forms; multimeric forms and aggregated forms.
